# EUROPEAN PATENT SPECIFICATION

(11) **EP 1 437 149 B1**
(45) Date of publication and mention of the grant of the patent: **14.08.2013**
(21) Application number: 02800014.9
(22) Date of filing: 25.09.2002
(51) Int. Cl.: A61L 27/56, A61L 27/48

(54) **COMPOSITE MATERIAL FOR TISSUE REGENERATION**
ZUSAMMENGESETZTES MATERIAL FÜR DIE GEWEBEREGENERATION
MATERIAU COMPOSITE DESTINE A LA REGENERATION TISSULAIRE

(30) Priority: 27.09.2001 JP 2001297888
(43) Date of publication of application: 14.07.2004
(73) Proprietor: NITTA GELATIN INC., Osaka-shi Osaka 556-0022 (JP); Tabata, Yasuhiko, Uji-shi, Kyoto 611-0024 (JP)
(72) Inventor: TABATA, Yasuhiko, Uji-shi, Kyoto 611-0024 (JP); HIRAOKA, Yosuke, Yao-shi, Osaka 581-0024 (JP); MANDAI, Yoshinobu, Yao-shi, Osaka 581-0024 (JP)
(74) Representative: Glawe, Delfs, Moll
(86) International application number: PCT/JP2002/009893
(87) International publication number: WO 2003/028782

(56) References cited:
- EP-A- 0 325 195
- WO-A1-98/00180
- JP-A- 3 023 864
- US-B1- 6 309 454
- DATABASE WPI Week 199111 Thomson Scientific, London, GB; AN 1991-077288 XP002543871 & JP 03 023864 A (GUNTER & ZIMMERMANN CONSTR DIV) 31 January 1991 (1991-01-31) & DATABASE EPODOC [Online] EUROPEAN PATENT OFFICE, THE HAGUE, NL; -& JP 03 023864 A 31 January 1991 (1991-01-31)
- DATABASE BIOSIS [Online] BIOSCIENCES INFORMATION SERVICE, PHILADELPHIA, PA, US; April 2001 (2001-04), LEE JONG-EUN ET AL: "Characterization of UV-irradiated dense/porous collagen membranes: Morphology, enzymatic degradation, and mechanical properties" XP002543869 Database accession no. PREV200100291579 & YONSEI MEDICAL JOURNAL, vol. 42, no. 2, April 2001 (2001-04), pages 172-179, ISSN: 0513-5796
- M.CHVAPIL: "Collagen Sponge: Theory and Practice of Medical Applications" J. BIOMED. MATER. RES., vol. 11, 1977, pages 721-741, XP002543868

## Description

### TECHNICAL FIELD

The present invention relates to a hybrid material for regeneration of living body tissue, which is, for example, used as a scaffold material of cells for regeneration of living body tissue.

### BACKGROUND ART

As to the scaffold material of cells usable for regeneration of living body tissue, requested are the following: 1) having affinity with cells; 2) being able to retain sufficient mechanical strength for a term needed to regenerate the tissue; 3) having biodegradation absorbability, and in order not to hinder the passage of the regeneration of the tissue, enabling degradation and disappearance rapidly in proportion with its progress; 4) being porous in order to make cells easily enter and to enable supply of oxygen and nutrition to the resultant entered cells. As to such a scaffold material, a spongy molded product including collagen that is a naturally-occurring polymer has been hitherto known. However, it has a demerit such that the mechanical strength is low, and when the spongy molded product is really buried in a living body or immersed in a cell suspension for cell inoculation, there is a problem such that its shape cannot be maintained. As a material for regeneration of living body tissue, which solves the problem of the mechanical strength, proposed are such as spongy molded products and nonwoven fabrics including biodegradable synthetic polymers (e.g. poly(lactic acid), poly(glycolic acid), or copolymers of these, or copolymers between these and ε-caprolactone). However, the material including only the biodegradable synthetic polymer has problems such that it is inferior in affinity with cells to collagen, and that at the same time its decomposing and disappearing term is also too long from the viewpoint of the regeneration of the tissue. In this way, the material solely including the collagen or the biodegradable synthetic polymers has difficulty in satisfying the above 1) to 3) at the same time.

Accordingly, as one method in compensation for both demerits, a hybrid material including collagen and a biodegradable synthetic polymer is developed in recent years. In such as JP-A-236688/1995, proposed is a hybrid material, in which at least one portion of a sponge material including collagen is covered with a biodegradable plastic. However, the synthetic polymer exists on a surface of the above hybrid material, and therefore the hybrid material is excellent in the mechanical strength, but its biocompatibility is yet still low, and there is room for improvement thereof. On the other hand, in *"*Artificial Internal Organs ", vol. 29 (2), 463 to 467 (2000), reported is a hybrid material obtained by a process including the steps of: immersing a sponge in a collagen solution, wherein the sponge is produced from a lactic acid-glycolic acid copolymer; and thereafter freeze-drying the resultant sponge. However, the hybrid material as obtained in this way has a high ratio of the synthetic polymer to the collagen, and the satisfactory performance is not obtained sufficiently with respect to affinity with cells, either. In addition, the hybrid material has problems such that the term as needed for degradation and absorption of the material in a living body is also too long from the viewpoint of the regeneration of the tissue.

JP-A-03023864 discloses tissue filler fillers comprising crosslinked collagen sponges and synthetic bioresorbable polymer fibres.

WO-A-9800180 discloses complexes comprising crosslinked collagen sponges and fibrous oxidized regenerated cellulose.

EP-A-1153622 discloses sponges comprising crosslinked collagen and fibrous oxidized regenerated cellulose.

### DISCLOSURE OF THE INVENTION

### OBJECT OF THE INVENTION

Accordingly, an object of the present invention is to provide: a hybrid material for regeneration of living body tissue, which is excellent in affinity with cells, and which can retain sufficient mechanical strength for a term needed to regenerate the tissue, and which simultaneously has moderate biodegradation absorbability which, in order not to hinder the process of tissue regeneration, enables degradation and disappearance rapidly in proportion with the process of tissue regeneration.

### SUMMARY OF THE INVENTION

Considering the aforementioned object, in a hybrid material comprising a naturally-occurring polymer (e.g. collagen) and a biodegradation-absorbable synthetic polymer, the present inventors diligently studied in order to control the content of the synthetic polymer low and further to effectively make the hybrid material retain mechanical strength. As a result, they found out that the aforementioned problems could be solved by making the biodegradation-absorbable synthetic polymer exist in a fibrous form and making the naturally-occurring polymer crosslinked, and then they attained the present invention.

That is to say, a hybrid material for regeneration of living body tissue, according to the present invention, is
a hybrid material for regeneration of living body tissue comprising (A) a sponge including a crosslinked naturally-occurring polymer and (B) biodegradation-absorbable synthetic polymer fibers, said hybrid material being characterized in
that said biodegradation-absorbable synthetic polymer fibers exist in the sponge,
that said biodegradation-absorbable synthetic polymer fibers are coated with a biodegradation-absorbable synthetic polymer having a melting point lower than the melting point of the biodegradation-absorbable synthetic polymer fibers for flocculating at an intersecting point, and
that the weight ratio of biodegradation-absorbable synthetic polymer/naturally-occurring polymer is in the range of 0.2 to 10.0.

### BRIEF DESCRIPTION OF THE DRAWINGS

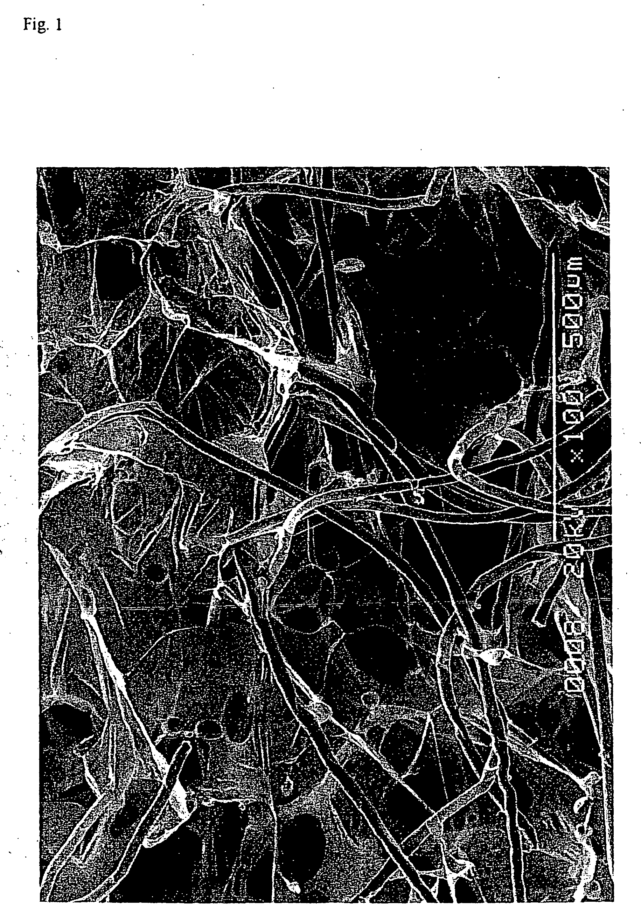

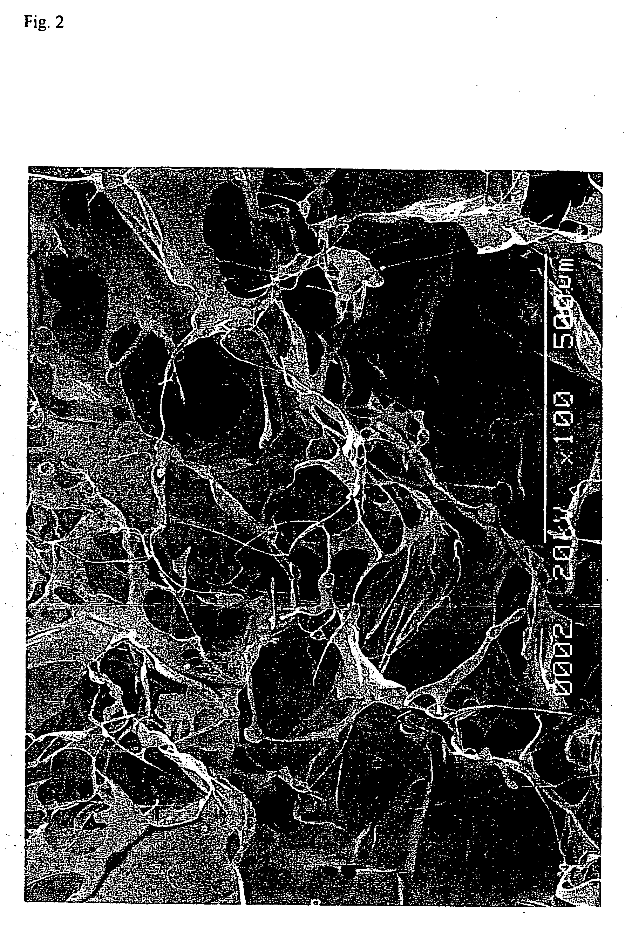

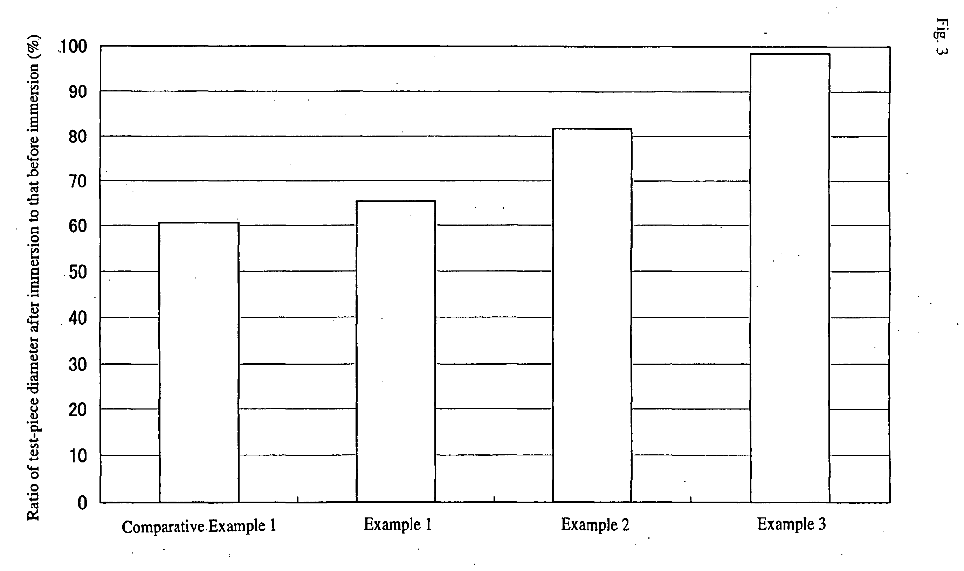

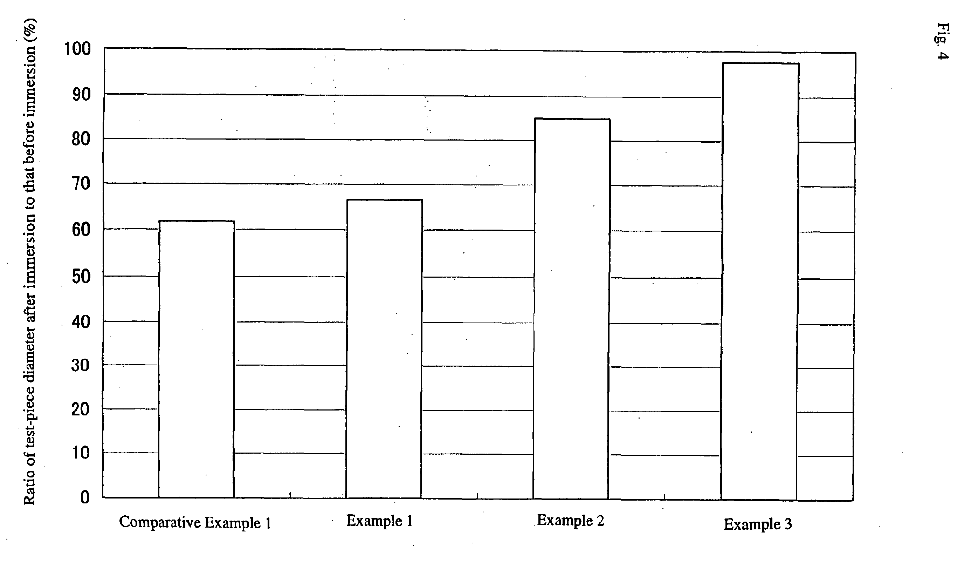

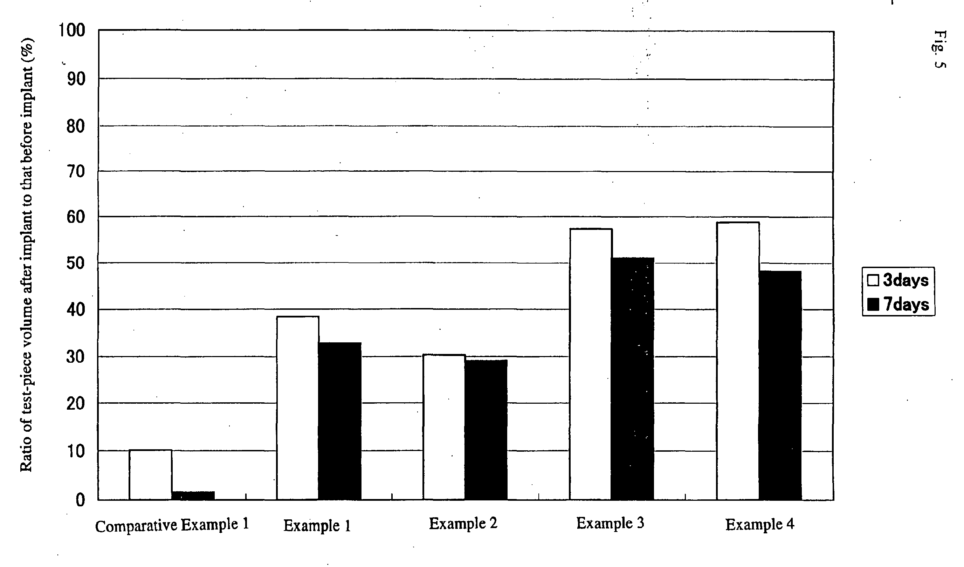

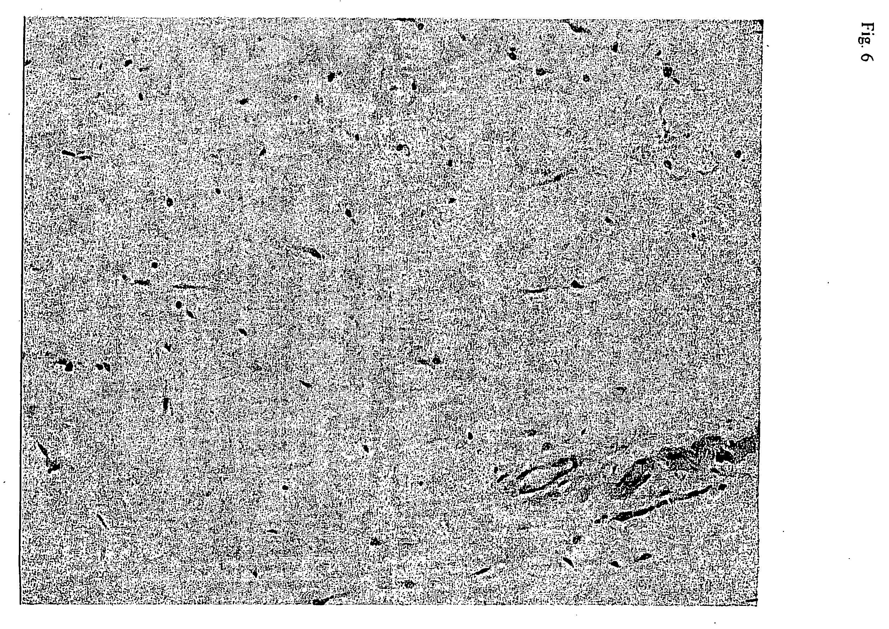

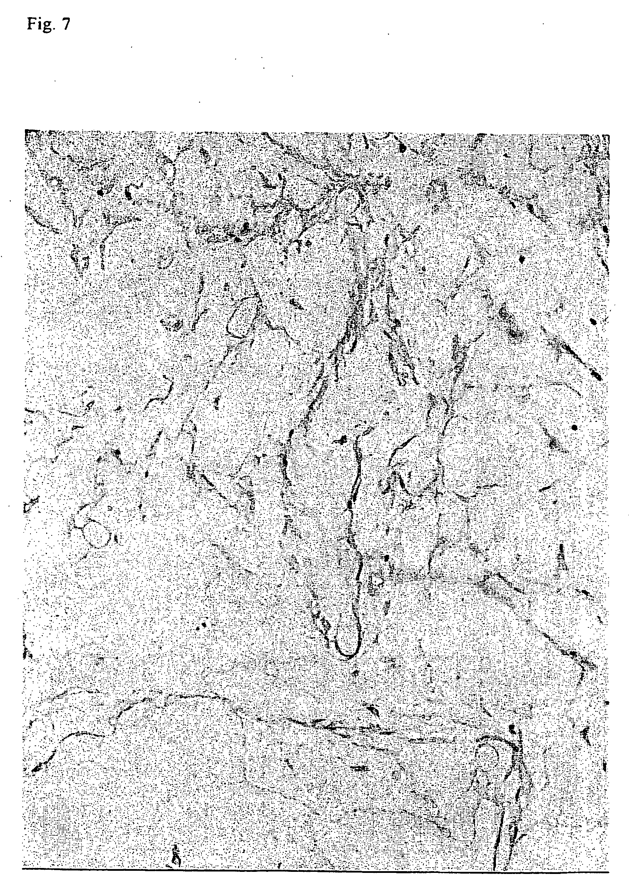

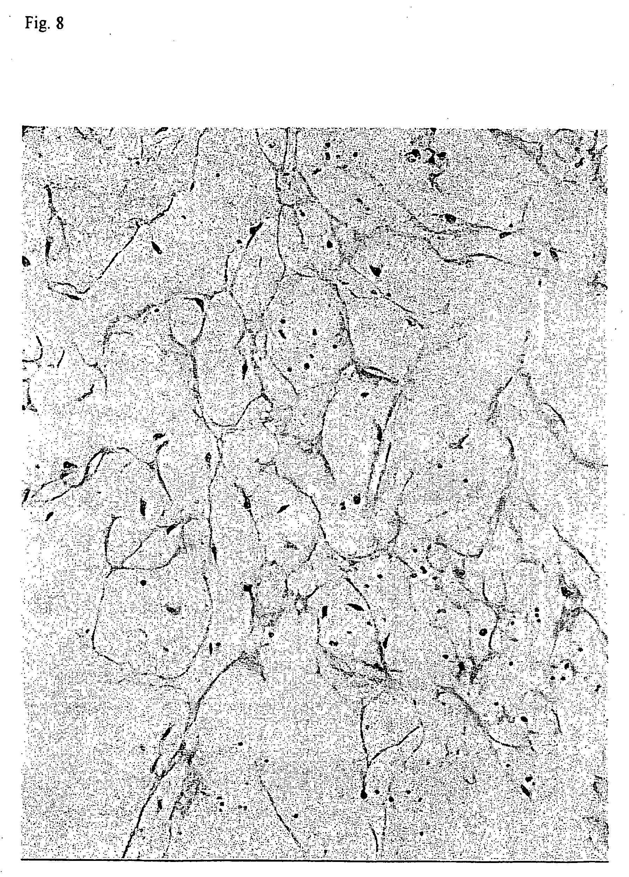

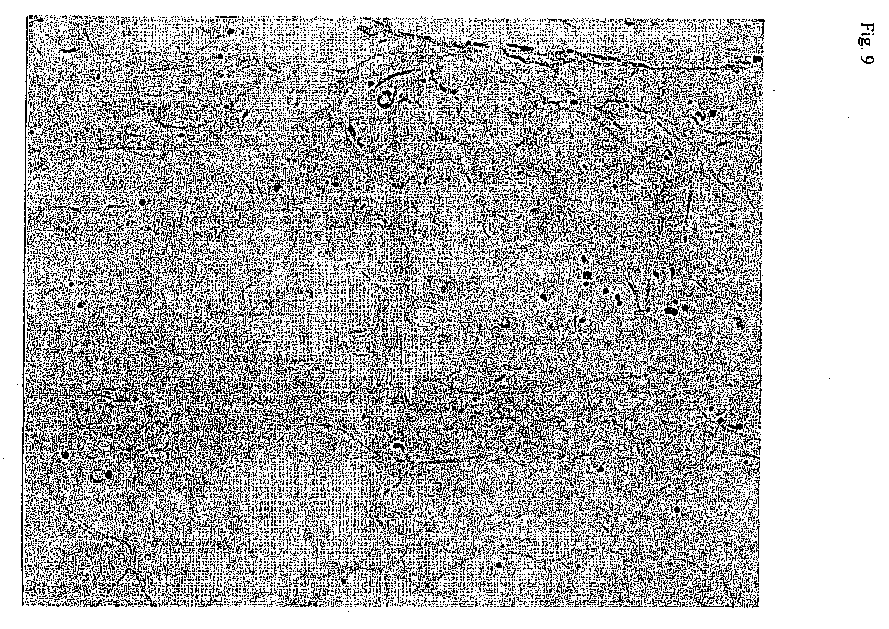

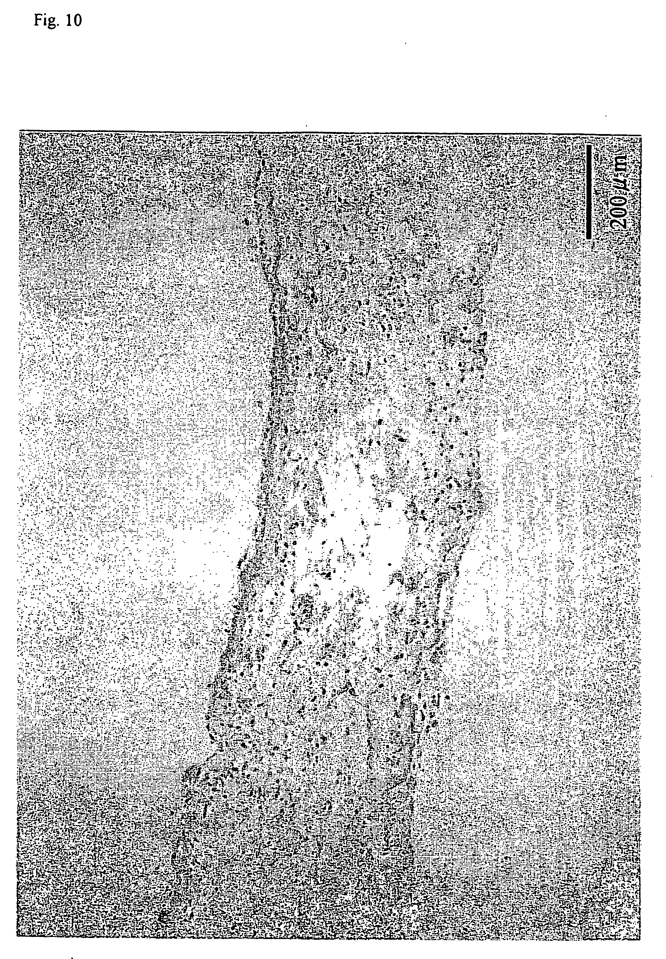

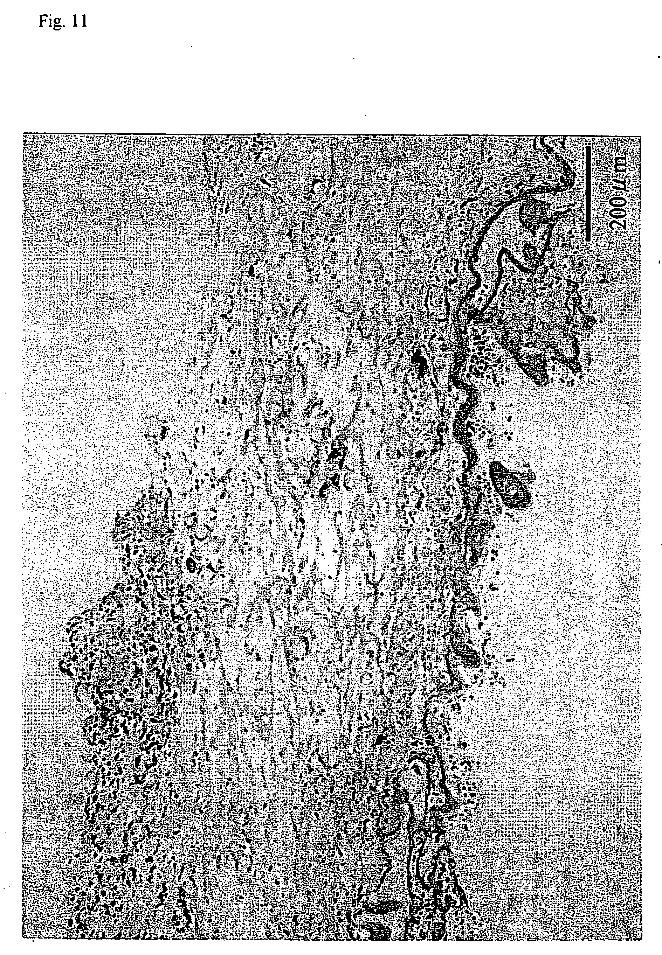

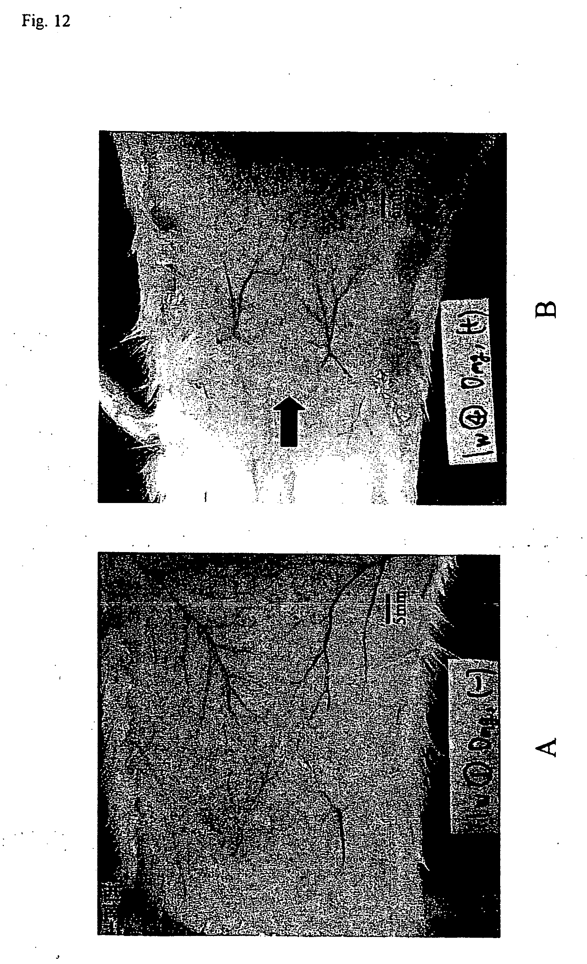

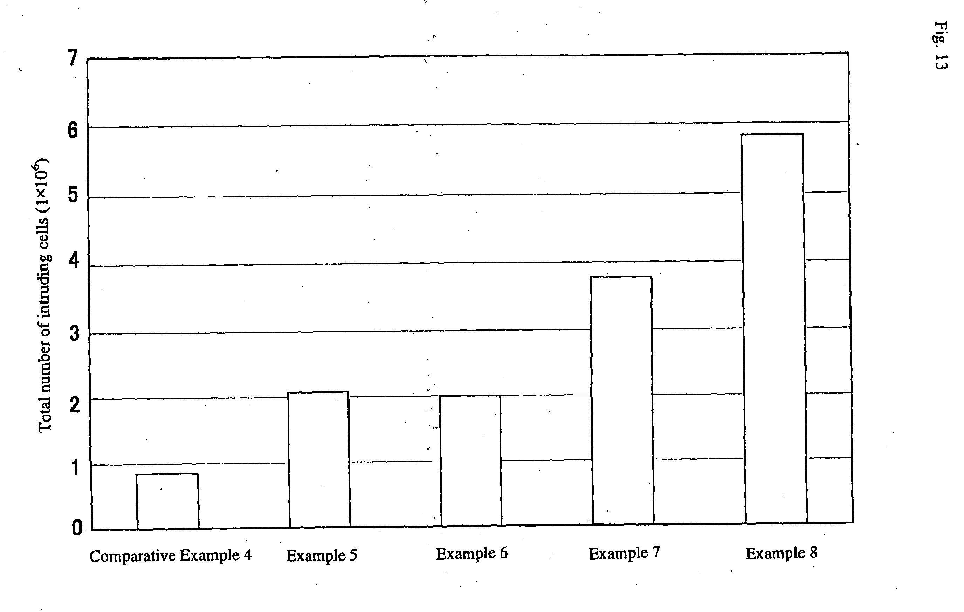

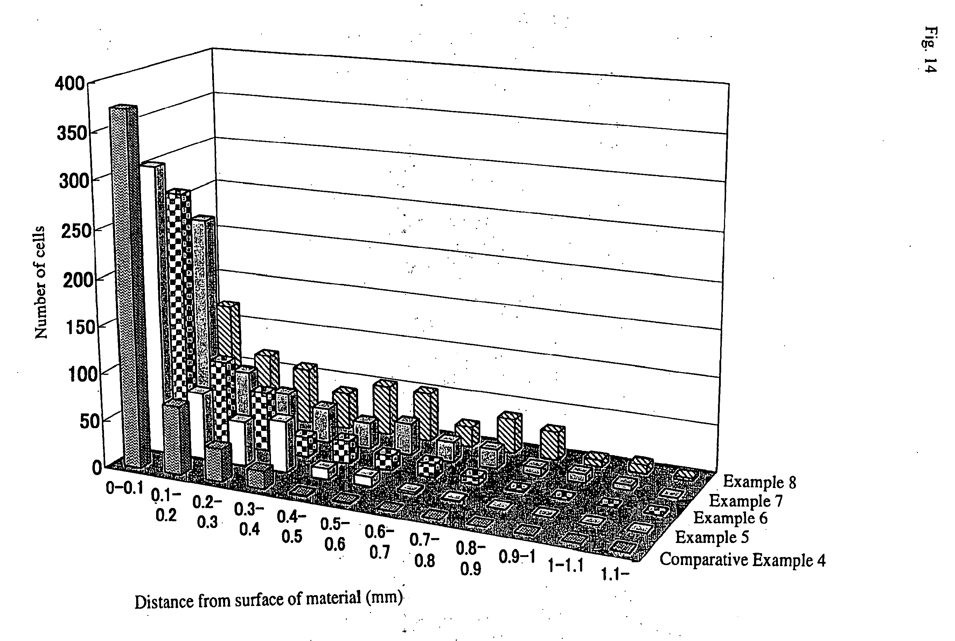

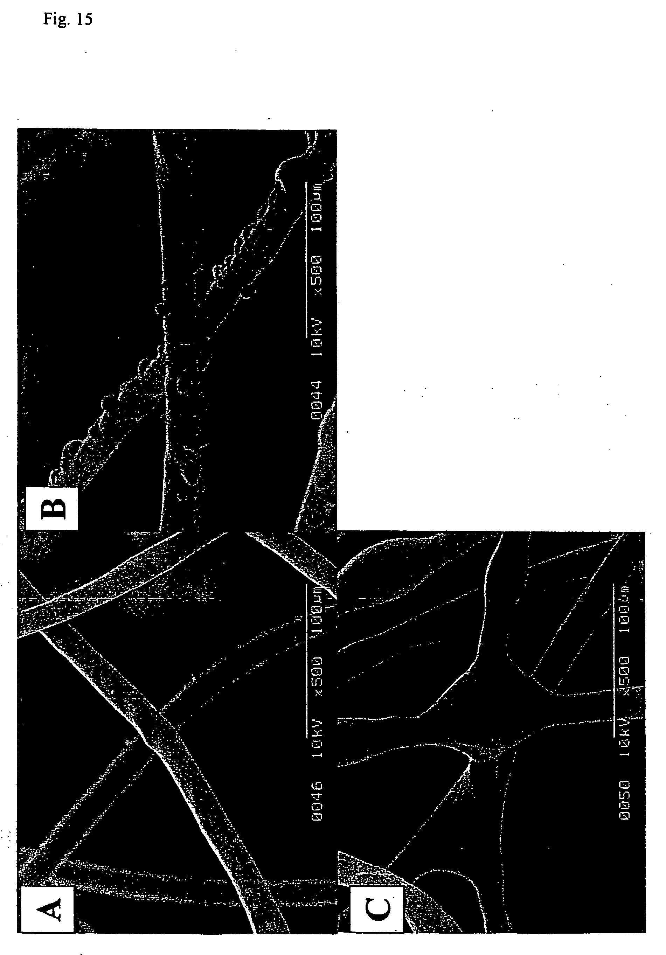

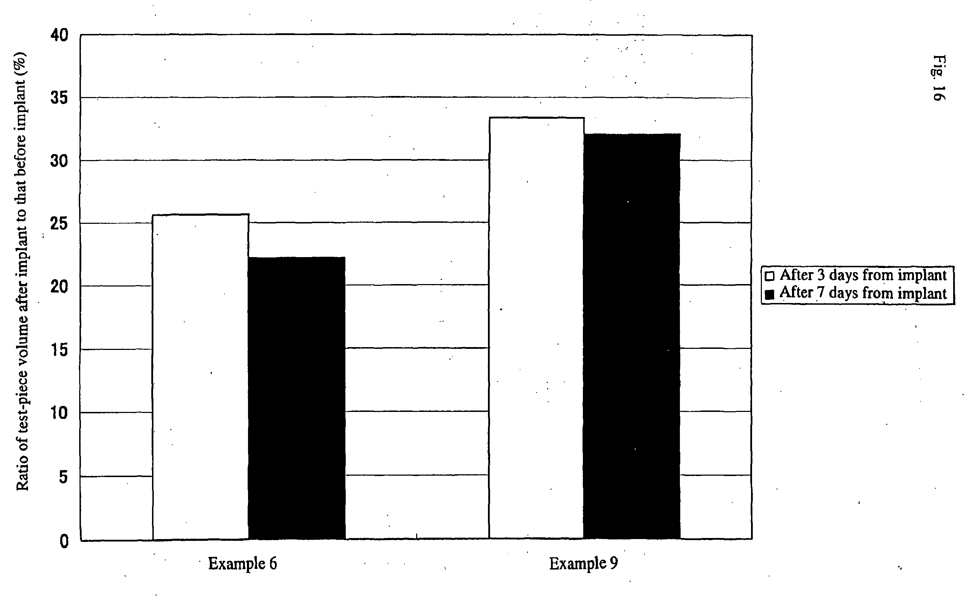

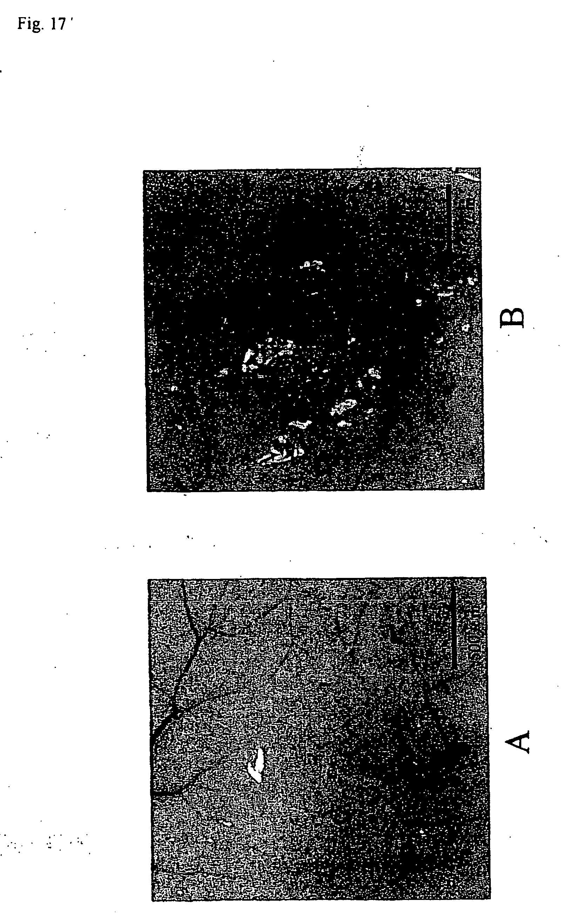

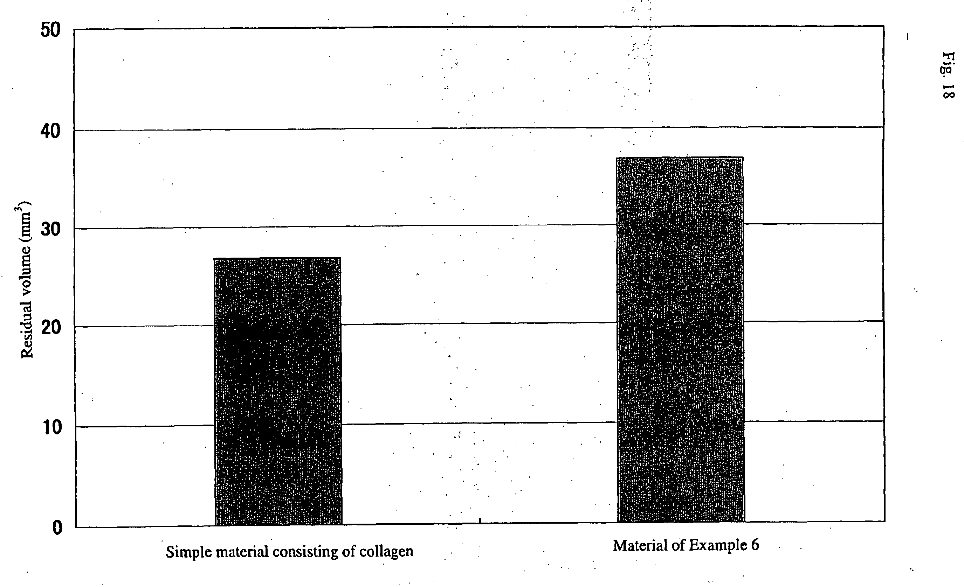

Fig. 1 is a view obtained by scanning-electron photomicrography of a section of a hybrid material as obtained in Example 1. Fig. 2 is a view obtained by scanning-electron photomicrography of a section of a material as obtained in Comparative Example 1. Fig. 3 is a graph showing a result of contraction test (in a case where cells are contained in a culture medium). Fig. 4 is a graph showing a result of contraction test (in a case where cells are not contained in a culture medium). Fig. 5 is a graph showing a result of mechanical strength in implant test 1 to under the back skin of a mouse. Fig. 6 is a view showing a H·E stain image in the evaluation of affinity of a material of Comparative Example 1 with cells in implant test 1 to under the back skin of a mouse. Fig. 7 is a view showing a H·E stain image in the evaluation of affinity of a material of Example 1 with cells in implant test 1 to under the back skin of a mouse. Fig. 8 is a view showing a H·E stain image in the evaluation of affinity of a material of Example 3 with cells of in implant test 1 to under the back skin of a mouse. Fig. 9 is a view showing a H·E stain image in the evaluation of affinity of a material of Example 4 with cells in implant test 1 to under the back skin of a mouse. Fig. 10 is a view showing a H·E stain image in the evaluation of affinity of a material of Comparative Example 2 with cells in implant test 2 to under the back skin of a mouse. Fig. 11 is a view showing a H·E stain image in the evaluation of affinity of a material of Example 2 with cells in implant test 2 to under the back skin of a mouse. Fig. 12 includes views obtained by photography of states of the remaining of materials during implant in implant test 2 to under the back skin of a mouse. Figs. 12A and 12B show a state of the remaining of a material of Comparative Example 3 and a state of the remaining of a material of Comparative Example 1, respectively. Fig. 13 is a graph showing a result of total number of cells intruding into a material in implant test 3 to under the back skin of a mouse. Fig. 14 is a graph showing a result of distance of intruding cells from a surface of a material in implant test 3 to under the back skin of a mouse. Fig. 15 includes views obtained by scanning-electron photomicrography of respective sections of a poly(glycolic acid) fiber (A), poly(glycolic acid) fiber (B) as coated with poly(lactic acid), and poly(glycolic acid) fiber (C) as coated with poly(lactic acid) and thereafter heat-treated at 195 °C for 30 minutes, in Example 9. Figs. 15A, 15B, and 15C show the poly(glycolic acid) fiber (A), the poly(glycolic acid) fiber (B), and poly(glycolic acid) fiber (C), respectively. Fig. 16 is a graph showing a result of mechanical strength in implant test 4 to under the back skin of a mouse. Fig. 17 includes views obtained by digital camera photography of appearance of samples in fat regeneration test under the back skin of a mouse. Figs. 17A and 17B show appearance of a sample including a simple material consisting of collagen and appearance of a sample including a material of Example 6, respectively. Fig. 18 is a graph showing a result of residual volume of a sample as taken out.

### DETAILED DESCRIPTION OF THE INVENTION

The hybrid material for regeneration of living body tissue, according to the present invention, can be obtained by a process including the steps of: immersing a fibrous biodegradation-absorbable synthetic polymer in a naturally-occurring polymer solution; freeze-drying the resultant mixture in the above immersing state; and thereafter subjecting the resultant dried product to crosslinking treatment.

There is no especial limitation on the naturally-occurring polymer in the present invention, and examples thereof include: proteins (e.g. collagen, gelatin, fibrin, gluten, and fibroin) and derivatives of these; poly(amino acids) and derivatives of these; polysaccharides (e.g. chitin, chitosan, hyaluronic acid, alginic acid, starch, and dextran) and derivatives of these; and mixtures including at least two kinds of these, and their hybrids produced by chemical bonding. Incidentally, the naturally-occurring polymers may be used either alone respectively or in combinations with each other.

In preferred modes of the present invention, the naturally-occurring polymer is favorably a collagen. There is no especial limitation on the collagen. Usable are collagen solutions of such as: publicly hitherto known acid-soluble collagens, enzyme-soluble collagen, base-soluble collagens, as obtained from a raw material such as animal bones and skins; or chemically modified collagens of these soluble collagens; or regenerated collagens as obtained by regenerating collagen fibers from the soluble collagens. Specific examples thereof include type-I collagens derived from pig skins, type-I collagens derived from pig tendons, type-II collagens derived from cattle nose gristles, and type-I collagens extracted from fishes.

When the collagen is used as the aforementioned naturally-occurring polymer, there is no especial limitation on the concentration of the collagen solution, but it is favorably set in the range of 0.1 to 4 weight %, more favorably 0.3 to 2 weight %.

In addition, when the collagen is used as the aforementioned naturally-occurring polymer, organic solvents having immiscibility with water can also be added to the aforementioned collagen solution in order that the pore size of the collagen sponge as obtained by effectively foaming the collagen solution will be set in an optimal range. Examples of such organic solvents include: halogenated hydrocarbons, such as chloroform, carbon tetrachloride, and methylene chloride; esters, such as ethyl acetate and ethyl propionate; aromatic hydrocarbons, such as benzene and toluene; aliphatic hydrocarbons, such as hexane and cyclohexane; and ethers, such as diethyl ether and diisopropyl ether. These may be used either alone respectively or in combinations with each other. Incidentally, when these organic solvents are added thereto, the organic solvent concentration after addition is favorably set not less than 2 weight % in order to display the effect sufficiently.

Furthermore, when the collagen is used as the aforementioned naturally-occurring polymer, various additives as hitherto used in the production of collagen sponges may be added to the aforementioned collagen solution, if necessary, in such a range as not to hinder the effects of the present invention. The biocompatibility can be enhanced more by adding such as: mucopolysaccharides, cell adhesion factors, cell growth factors, cytokinins, and chemokinins; or proteins or peptides having physiological activity that these substances have.

Examples of the biodegradation-absorbable synthetic polymer in the present invention include poly(glycolic acid), poly(lactic acid), poly-ε-caprolactone, polydioxanone, poly(β-malic acid), poly(ortho esters), polydiaminophosphazene, and copolymers of these. Among these, the poly(glycolic acid), poly(lactic acid), poly(ε-caprolactone), or copolymers of these are favorable, particularly in view of having biodegradation absorbability that is suitable for a term for general regeneration of tissue. Incidentally, the biodegradation-absorbable synthetic polymers may be used either alone respectively or in combinations with each other.

It is important that the aforementioned biodegradation-absorbable synthetic polymer is fibrous. The presence of the biodegradation-absorbable synthetic polymers in a fibrous form can retain the mechanical strength sufficiently with a small content. Specifically, the above fiber may be a long fiber or a short fiber. However, the fiber is favorably entangled sufficiently in such as a state of unfastening nonwoven fabrics. In addition, there is no especial limitation on the diameter of the fiber, either. Favorably usable are fibers having diameters of such as 1 to 100 µm.

The fiber of the aforementioned biodegradation-absorbable synthetic polymer has sites to bond its adjacent fibers together. Thereby, the strength of the biodegradation-absorbable synthetic polymer fiber is reinforced, the more excellent mechanical strength can be given to the resultant hybrid material. In addition, thereby, the mechanical strength can sufficiently be given to the hybrid material as obtained by using the biodegradation-absorbable synthetic polymer fiber with a smaller quantity.

There is no especial limitation on a method for forming the sites to bond adjacent fibers of the aforementioned biodegradation-absorbable synthetic polymer together. The method is carried out by a process including the steps of: coating the aforementioned fibrous biodegradation-absorbable synthetic polymer with a biodegradation-absorbable synthetic polymer having a lower melting point than a polymer used as the aforementioned fibrous biodegradation-absorbable synthetic polymer (hereinafter, referred to as a low-melting-point polymer), such as poly(lactic acid), copolymers of lactic acid and glycolic acid, poly-ε-caprolactone, copolymers of lactic acid and ε-caprolactone; and thereafter heat-treating the resultant coated product under conditions such that only the above coated low-melting-point polymer is melt. The fibrous biodegradation-absorbable synthetic polymer is subjected to such treatment, and thereby only the low-melting-point polymer as coated on the fiber is melted, and this melted low-melting-point polymer is flocculated at an intersecting point with the result that the sites to bond its adjacent fibers together are formed. Specifically, the coating of the aforementioned low-melting-point polymer may be carried out, for example, by a process including the steps of: preparing a solution by dissolving the above low-melting-point polymer in a solvent such as chloroform, ethyl acetate, and dioxane; spraying the above solution onto a surface of the fiber with such as a sprayer; and thereafter removing the aforementioned solvent by such as freeze-drying. Incidentally, then, the coating amount of the aforementioned low-melting-point polymer is favorable set in the range of 0.1 to 50 weight % relative to the fibrous biodegradation-absorbable synthetic polymer. In addition, the condition during the heat treatment is not especially limited if it is such as a condition such that only the aforementioned low-melting-point polymer is melt. For example, the heat treatment may be carried out under a vacuum pressure of lower than 0.1 to 0.5 torr at 160 to 250 °C for 5 to 60 minutes.

The content of the biodegradation-absorbable synthetic polymer is set so that the ratio of biodegradation-absorbable synthetic polymer/naturally-occurring polymer (weight ratio) will be in the range of 0.2 to 10.0. Since the biodegradation-absorbable synthetic polymer displays the fibrous form in the present invention, the mechanical strength can be retained sufficiently even if the content of the biodegradation-absorbable synthetic polymer is such a small amount as the aforementioned range. Then, the small content of the biodegradation-absorbable synthetic polymer causes the excellent affinity with cells, and at the same time it can be caused to rapidly decompose and disappear after the regeneration of the tissue. When the collagen is used as the aforementioned naturally-occurring polymer, the amount of the biodegradation-absorbable synthetic polymer is really set favorably in the range of 0.01 to 10 weight %, more favorably 0.4 to 1 weight %, relative to the aforementioned collagen solution (in a state before adding the aforementioned organic solvent and various additives).

The freeze-drying may be carried out, for example, in a state where the biodegradation-absorbable synthetic polymer fibers are impregnated with a solution of the aforementioned naturally-occurring polymer by adding this solution into a receptacle containing the aforementioned fibrous biodegradation-absorbable synthetic polymer. Then, it is favorable that the naturally-occurring polymer solution as added to the receptacle is sufficiently foamed with such as a conventional homogenizer.

The freeze-drying method may be carried out according to a conventional method. For example, the temperature during the freeze-drying may be set in the range of -4 to -196°C.

It is important to successively subject the sponge resultant from the freeze-drying to crosslinking treatment. Since the naturally-occurring polymer such as the collagen is crosslinked in the above crosslinking treatment, the mechanical strength can be retained sufficiently even if the content of the biodegradation-absorbable synthetic polymer is small.

There is no especial limitation on the crosslinking-treating method, and adopted may be such as publicly hitherto known chemical crosslinking methods, heat-dehydration crosslinking methods under vacuum, crosslinking methods by ultraviolet irradiation. Specifically, when the chemical crosslinking methods are carried out, crosslinking agents such as glutaraldehyde may be used. When the heat-dehydration crosslinking methods are carried out under vacuum, the methods may be carried out under conditions: 100 to 150°C and about 2 to about 120 hours. When the crosslinking methods are carried out by ultraviolet irradiation, the irradiation of ultraviolet ray of such as 254 nm may be carried out for about 1 minute to about 24 hours.

The present invention hybrid material for regeneration of living body tissue as obtained in this way has a structure such that the fibrous biodegradation-absorbable synthetic polymer is randomly buried in the crosslinked microporous sponge including the naturally-occurring polymer.

The hybrid material for regeneration of living body tissue, according to the present invention, is excellent in affinity with cells, and can retain sufficient mechanical strength for a term needed to regenerate the tissue, and simultaneously has moderate biodegradation absorbability which enables degradation and disappearance rapidly after the regeneration of the tissue. Therefore, the hybrid material is useful as a scaffold material when the cell proliferation and differentiation are carried out in order to regenerate deficient portions of such as soft tissue (e.g. skins, muscles, and fats), nerve tissue, gristle tissue, bone tissue, substantial tissue (e.g. livers and kidneys), digestive organs (e.g. gullets, and stomach and intestines), tracheas, urinary organs (e.g. bladders, ureters, and urethra), tooth-around tissue, tendons, and ligaments. In addition, the hybrid material for regeneration of living body tissue, according to the present invention, is also usable as a culture-scaffold material for in-vitro proliferation and differentiation of various cells such as stem cells.

### DETAILED DESCRIPTION OF THE PREFERRED EMBODIMENTS

### <Example1> Comparative example, not forming part of the invention)

An amount of 3 mg of nonwoven fabric including poly(glycolic acid) was cut off and uniformly disentangled with tweezers to make it fibrous, and then put in an aluminum cap (20 mmφ, 34 mmH) coated with silicone ("Sigmacoat" produced by SIGMA). Next, chloroform was added to 1.5 g of 0.3 weight % type-I collagen solution derived from pig skins so that the chloroform concentration would be about 5 weight %, and the resultant solution was stirred with a homogenizer equipped with a generator shaft at 12,000 rpm for 3 minutes, and thereafter the above solution was added to the aforementioned aluminum cap. Subsequently, the content of the above aluminum cap was frozen at -80°C for 12 hours, and thereafter freeze-dried under vacuum of lower than 0.1 torr for 24 hours. Thereafter, the resultant dried product was heat-dehydration-crosslinked at 140°C under vacuum of lower than 0.1 torr for 12 hours, thus obtaining a hybrid material, in which the ratio of poly(glycolic acid)/collagen was 0.67 (weight ratio).

### <Examples 2 to 4> (Comparative examples, not forming part of the invention)

A hybrid material, according to the present invention, was obtained in the same way as of Example 1 except that the weight of the nonwoven fabric including the poly(glycolic acid) was each changed to 6 mg in Example 2, 12 mg in Example 3, and 24 mg in Example 4.

### <Comparative Example 1>

Chloroform was added to 1.5 g of 0.3 weight % type-I collagen solution derived from pig skins so that the chloroform concentration would be about 5 weight %, and the resultant solution was stirred with a homogenizer equipped with a generator shaft at 12,000 rpm for 3 minutes, and thereafter the above solution was put in an aluminum cap (20 mmφ, 34 mmH) coated with silicone ("Sigmacoat" produced by SIGMA). Subsequently, the content of the above aluminum cap was frozen at -80°C for 12 hours, and thereafter freeze-dried under vacuum of lower than 0.1 torr for 24 hours. Thereafter, the resultant dried product was heat-dehydration-crosslinked at 140°C under vacuum of lower than 0.1 torr for 12 hours, thus obtaining a comparative simple material consisting of collagen.

### <Comparative Example 2>

A comparative hybrid material was obtained in the same way as of Example 2 except that the heat-dehydration crosslinking was not carried out.

### <Comparative Example 3>

A comparative simple material consisting of collagen was obtained in the same way as of Comparative Example 1 except that the heat-dehydration crosslinking was not carried out.

### (Observation with scanning-electron microscope):

The section of materials as obtained in Example 1 and Comparative Example 1 was observed with a scanning electron microscope. The section of Example 1 and that of Comparative Example 1 are shown in Figs. 1 and 2, respectively.

From Figs. 1 and 2, the fibrous biodegradation-absorbable synthetic polymer is found in the present invention hybrid material, and it is understood that there is no difference in pore size whether the biodegradation-absorbable synthetic polymer fiber exists or not

### (Compression test):

The following compression test was carried out by using the materials as obtained in Example 4 and Comparative Example 1.

The material as obtained was cut to obtain a column having a diameter of 18 mm and a height of 3 mm, and the stress-distortion curve was obtained when the column was compressed to 50 % of the height with a compression test machine ("Autograph AGS-10kND" produced by Shimadzu Seisakusho) at a speed of 1 mm/minute. Then, the modulus of compressional elasticity was determined from the above curve.

As a result above, the material of Example 4 had a modulus of compressional elasticity of 39.4 MPa, but that of Comparative Example 1 had a modulus of compressional elasticity of 6.6 MPa. From this fact, it would be suggested that the hybrid material comprising the biodegradation-absorbable synthetic polymer fiber can display more resistance to higher compression than the simple material consisting of collagen even when the implant is carried out.

### (In-vitro contraction test):

The materials as obtained in Examples 1 to 3 and Comparative Example 1 were each processed to obtain a columnar test piece having a diameter of 15 mm and a height of 3 mm, and this was immersed in 70 % ethanol for 3 minutes, and thereafter immersed in an Eagle's MEM culture medium (containing 10 % of FCS) three times. After the culture medium was removed, the test piece was put in a plate, and 1 ml of Eagle's MEM culture medium (containing 10 % of FCS) including L929 fibroblast (2× 10⁶ cells/ml) was added thereto, and the resultant mixture was incubated at 37 °C. Then, the diameter of the test piece was measured after 6 hours, and the ratio of the test-piece diameter after immersion to that before immersion was calculated. The result is shown in Fig. 3. Incidentally, the result was represented by an average value as obtained when the test for each material was carried out three by three.

On the other hand, the diameter of the test piece was measured after 6 hours in the same way as of the above, except that an Eagle's MEM culture medium (containing 10 % of FCS) not including L929 fibroblast (2× 10⁶ cells/ml) was used instead of that including L929 fibroblast (2× 10⁶ cells/ml), and then the ratio of the test-piece diameter after immersion to that before immersion was calculated. The result is shown in Fig. 4. Incidentally, the result was represented by an average value as obtained when the test for each material was carried out three by three.

From the results of Figs. 3 and 4, it was apparent that: the simple material consisting of collagen was greatly contracted by the immersion in the culture medium whether the cells exist in the culture medium or not, but the contraction of the present invention hybrid material comprising the biodegradation-absorbable synthetic polymer fiber was controlled small. This is supposed because the biodegradation-absorbable synthetic polymer fiber fills the role of skeleton in the material sponge. In addition, when the content of the biodegradation-absorbable synthetic polymer fiber was the same, no contraction difference was observed whether the cells existed in the culture medium or not.

### (In-vitro bio-affinity test):

The materials as obtained in Example 2 and Comparative Example 1 were each processed to obtain a columnar test piece having a diameter of 15 mm and a height of 3 mm, and this was immersed in 70 % ethanol for 3 minutes, and thereafter immersed in an Eagle's MEM culture medium (containing 10 % of FCS) three times. After the culture medium was removed, the test piece was put in a plate, and 0.5 ml of Eagle's MEM culture medium (containing 10 % of FCS) including L929 fibroblast (2×10⁶ cells/ml) was added thereto, and the resultant mixture was incubated at 37 °C. Then, the number of the cells as adhered after 6 hours was measured by determining DNA of the cells.

As a result above, the number of the material of Example 2 was 22,109, and that of the material of Comparative Example 1 was 19,908. Accordingly, no residual error was found between both of them. These results show that the cell affinity of the collagen sponge is not changed whether the biodegradation-absorbable synthetic polymer fiber exists in the culture medium or not.

From the above in-vitro test results, it is understood that, if the sponge is contracted in the stage of adding a cell suspension to the sponge, it is difficult to utilize the material as a culture-scaffold material for cells. In comparison with this, it was apparent that such contraction was not found in the material, and that the material had the same cell affinity as original collagens and was also excellent as an in-vitro culture scaffold for cells.

### (Implant test 1 to under the back skin of a mouse):

The materials as obtained in Examples 1 to 4 and Comparative Example 1 were EOG-sterilized, and thereafter they were each cut to obtain a test piece having a size of 5 mm x 5 mm. A mouse (ddy, ♀, supplied from Shimizu Jikken Zairyo) was cut at its back portion partially, and therefrom two test pieces aforementioned were buried under the back skin of a mouse, and thereafter the portion was sutured. Then, the test pieces were taken out after 3 and 7 days, and the mechanical strength and affinity with cells were evaluated in the following way.
1) Mechanical strength; The volume of the test piece as taken out was measured, and the ratio of the test-piece volume after implant to that before implant was calculated. The result is shown in Fig. 5. Incidentally, the result was represented by an average value as obtained when the test for each material was carried out three by three.

From the result of Fig. 5, it was apparent that: compared with the simple material consisting of collagen of which the volume was decreased to about 10 % of the volume before implant for 3 days and to smaller than 2 % of the volume before implant for 7 days, the decrease of the volume could be significantly suppressed in any hybrid material comprising the biodegradation-absorbable synthetic polymer fiber.
2) Affinity with cells; The test piece as taken out after 7 days (except for Example 2) was cut at a central site of the test piece and stained with hematoxylin·eocin, and then the number of cells as observed on their sections was measured. As a result, the average value as obtained when carrying out the measurement for each material three by three was 696.4 for the material of Comparative Example 1, 716.2 for that of Example 1, 1,680.9 for that of Example 3, and 1,768.1 for that of Example 4. The hematoxylin·eocin (H·E) stain images of the materials of Comparative Example 1, Example 1, Example 3, and Example 4 as used then are shown in Fig.6, Fig. 7, Fig. 8, and Fig. 9, respectively.

From the above result of the number of cells, it was apparent that the number of cells intruding into the material was increased by comprising the biodegradation-absorbable synthetic polymer fiber, and that the number was increased in a proportion with the increase of the fiber content. It is thought that this is caused because the contraction of the material is suppressed and the residual volume of the collagen sponge is increased by comprising the biodegradation-absorbable synthetic polymer fiber.

### (Implant test 2 to under the back skin of a mouse):

The materials as obtained in Example 2 and Comparative Example 2 were used, and test pieces were produced and implanted to under the back skin of a mouse in the same way as of the above implant test 1. Then, the test piece was taken out after 14 days, and the mechanical strength and affinity with cells were evaluated in the following way.
1) Mechanical strength; the one-edge length of the test piece as taken out was measured, and the mechanical strength was evaluated. As a result, the average value as obtained when carrying out the measurement for each material three by three was 0.93 mm for the material of Example 2, and 0.40 mm for the uncrosslinked material of Comparative Example 2.
2) Affinity with cells; The test piece as taken out was cut at a central site of the test piece and stained with hematoxylin·eocin, and then the number of cells as observed on their sections was measured, and the number of cells per a unit area was calculated. As a result, the average value as obtained when carrying out the measurement for each material three by three was 601.5 pieces/mm² for the material of Comparative Example 2 and 817.0 pieces/mm² for that of Example 2. In addition, the hematoxylin·eocin (H·E) stain images of the materials of Comparative Example 2 and Example 2 are shown in Fig. 10 and Fig. 11, respectively.

From the results of Figs. 10 and 11 and the above number of cells, as to the uncrosslinked material of Comparative Example 2, the residual buried collagen was not observed for 14 days after implant by the H·E stain image, and the collagen decomposed and disappeared from the buried site. It is thought that the collagen sponge cannot be a scaffold for cell proliferation and differentiation in vivo because the collagen has disappeared so fast. On the other hand, it was confirmed that: as to the crosslinked material, the buried collagen remained even at this time, and the cells intruded into the sponge and proliferated.

On the other hand, the materials as obtained in Comparative Examples 1 and 3 were used, and test pieces were produced and implanted to under the back skin of a mouse in the same way as of the above implant test 1, and the state of remaining of the materials under the back skin of a mouse was observed after 7 days. The view obtained by photographing the state of remaining of the material of Comparative Example 3 and the view obtained by photographing the state of remaining of the material of Comparative Example 1 are shown in Figs. 12A and 12B, respectively. As a result, as shown in Fig. 12 A, the uncrosslinked material of Comparative Example 3 completely disappeared after 7 days from implant. However, as to the crosslinked material of Comparative Example 1, it could be confirmed that the collagen sponge maintained its shape and remained at an arrowed portion in Fig. 12B. It is difficult to think that the inclusion of the biodegradation-absorbable synthetic polymer fiber has an influence upon the decomposability of the collagen sponge itself, and it would be thought that the uncrosslinked collagen sponge decomposes within at least 7 days.

From the result of the above implant test 2 to under the back skin of a mouse, it would be thought that: in the case of aiming at the regeneration of the living body tissue, if the material remains in vivo without decomposing and being absorbed for at least about 7 to about 14 days, the material is unsuitable as a scaffold material for cell proliferation and differentiation. It was shown that the crosslinking treatment of the collagen sponge was essential for achieving this condition.

### <Example 5> (Comparative example, not forming part of the invention)

An amount of 1.5 mg of nonwoven fabric including poly(glycolic acid) was cut off and uniformly disentangled with tweezers to make it fibrous, and then put in a 24-well plate (coaster 3526, corning, NY). Next, 0.75 g of 0.3 weight % type-I collagen solution derived from pig skins was added to the aforementioned 24-well plate. Subsequently, the content of the above 24-well plate was frozen at -80 °C for 12 hours, and thereafter freeze-dried under vacuum of lower than 0.1 torr for 24 hours. Thereafter, the resultant dried product was heat-dehydration-crosslinked at 140 °C under vacuum of lower than 0.1 torr for 12 hours, thus obtaining a hybrid material, in which the ratio of poly(glycolic acid)/collagen was 0.67 (weight ratio).

### <Examples 6 to 8> (Comparative examples, not forming part of the invention)

A hybrid material was obtained in the same way as of Example 5 except that the weight of the nonwoven fabric including the poly(glycolic acid) was each changed to 3 mg in Example 6, 6 mg in Example 7, and 12 mg in Example 8.

### <Comparative Example 4>

An amount of 0.75 g of 0.3 weight % type-I collagen solution derived from pig skins was put in a 24-well plate (coaster 3526, corning, NY). Subsequently, the content of the above 24-well plate was frozen at -80°C for 12 hours, and thereafter freeze-dried under vacuum of lower than 0.1 torr for 24 hours. Thereafter, the resultant dried product was heat-dehydration-crosslinked at 140 °C under vacuum of lower than 0.1 torr for 12 hours, thus obtaining a comparative simple material consisting of collagen.

### (Implant test 3 to under the back skin of a mouse):

The materials as obtained in Examples 5 to 8 and Comparative Example 4 were used, and test pieces were produced and implanted to under the back skin of a mouse in the same way as of the above implant test 1. Then, the test piece was taken out after 24 hours, and the total number of cells intruding into the material and the distance of the intruding cells from a surface of the material were evaluated as indexes for judgment of the affinity with cells in the following way.
1) Total number of cells intruding into material; The test piece as taken out after 24 hours was washed with PBS (phosphoric acid buffer solution) and thereafter the DNA assay was carried out to measure the total number of cells in the material. The number of cells was determined by calculation from a standard curve calculated by L929 fibroblast.

As a result above, the number of cells was 2,084,938 for the material of Example 5, 2,000,960 for the material of Example 6, 3,777,916 for the material of Example 7, 5,825,916 for the material of Example 8, and 849,226 for the material of Comparative Example 4. These results are graphed in Fig. 13. From these results, it was apparent that: the number of cells intruding into the material was increased by including the biodegradation-absorbable synthetic polymer fiber, and was increasing in proportion to the increase of the fiber content.
2) Distance of intruding cells from surface of material; The test piece as taken out after 24 hours was fixed with 4 % formalin solution and stained with hematoxylin·eocin. Then, at least two pieces of tissue as oriented in different sites were observed with Image-Pro Plus 3.01 (Silver Spring, MD), and the distance from the surface was measured as to 500 cells as optionally selected. The result is shown in Fig. 14.

From the result of Fig. 14, in the case of the simple material consisting of collagen, 99 % of the cells exist within 0.4 mm from the surface of the material, and it is apparent that the cells are difficult to intrude as far as the inner material. Compared with this, in the case of the hybrid material including the biodegradation-absorbable synthetic polymer fiber, the cells intrude as far as the inner material. In addition, it was apparent that the intruding distance increased and the cells intruded as far as the much inner material in proportion to the increase of the fiber content.

From the above result of the implant test 3 to under the back skin of a mouse, it was confirmed that: in the case of aiming at the regeneration of the living body tissue, the material was significantly more excellent in respect of the in-vivo intrusion of cells than the simple material consisting of collagen.

### <Example 9>

An amount of 3.0 mg of nonwoven fabric including poly(glycolic acid) was cut off and uniformly disentangled with tweezers to make it fibrous, thus obtaining a molded poly(glycolic acid) fiber (A) in a columnar shape (having a radius of about 8 mm and a height of about 3 mm). To a sprayer (YT-03, TORICON), added was 5 weight % poly(lactic acid) solution as obtained by dissolving poly(lactic acid) (having a molecular weight of 20,000, WAKO) in chloroform, and it was sprayed onto the surface of the aforementioned fibrous structure from a distance of 15 cm for some seconds. Thereafter, the chloroform was removed by freeze-drying, thus obtaining a poly(glycolic acid) fiber (B) coated with 20 weight % of the poly(lactic acid) relative to the poly(glycolic acid). Next, the above poly(glycolic acid) fiber (B) was heat-treated under vacuum of lower than 0.1 torr at 195 °C for 30 minutes, thus obtaining a poly(glycolic acid) fiber (C) coated with the poly(lactic acid) and thereafter heat-treated.

This poly(glycolic acid) fiber (C) was put in a 24-well plate (coaster 3526, corning, NY). Next, 0.75 g of 0.3 weight % type-I collagen solution derived from pig skins was added to the aforementioned 24-well plate. Subsequently, the content of the above 24-well plate was frozen at -80°C for 12 hours, and thereafter freeze-dried under vacuum of lower than 0.1 torr for 24 hours. Thereafter, the resultant dried product was heat-dehydration-crosslinked at 140 °C under vacuum of lower than 0.1 torr for 12 hours, thus obtaining a hybrid material according to the present invention, in which the ratio of poly(glycolic acid)/collagen was 1.33 (weight ratio).

Incidentally, the respective sections of the above poly(glycolic acid) fiber (A), poly(glycolic acid) fiber (B) coated with the poly(lactic acid), and poly(glycolic acid) fiber (C) coated with the poly(lactic acid) and thereafter heat-treated were observed with a scanning electron microscope. The result is shown in Fig. 15. In Fig. 15, the views A, B and C show the poly(glycolic acid) fiber (A), the poly(glycolic acid) fiber (B), and the poly(glycolic acid) fiber (C), respectively. From the result of Fig. 15, it is apparent that sites to bond its adjacent fibers together are formed in the poly(glycolic acid) fiber (C), and it was shown that: the heat treatment after coating with the poly(lactic acid) made only the coated poly(lactic acid) melt and solidify, and could form the sites to bond its adjacent fibers together.

### (Implant test 4 to under the back skin of a mouse):

The materials as obtained in Example 9 and Example 6 (in the same way as of Example 9 except that the heat treatment procedure was not carried out after coating the poly(glycolic acid) fiber with the poly(lactic acid)) were used, and test pieces were produced and implanted to under the back skin of a mouse in the same way as of the above implant test 1. Then, the test pieces were taken out after 3 and 7 days, and the mechanical strength was evaluated in the same way as of the implant test 1 to under the back skin of a mouse. The result is shown in Fig. 16.

From the result of Fig. 16, it was apparent that the material comprising the poly(glycolic acid) fiber having the sites to bond its adjacent fibers together was larger in respect of the residual volume after the implant than the material comprising the poly(glycolic acid) fiber not having the sites to bond its adjacent fibers together. This shows that the mechanical strength of the material comprising the poly(glycolic acid) fiber having the sites to bond its adjacent fibers together is much higher than that of the material comprising the poly(glycolic acid) fiber not having the sites to bond its adjacent fibers together.

### (Fat regeneration test under the back skin of a mouse):

The materials as obtained in Example 6 and the simple material consisting of collagen (collagen sponge "Pelunac" produced by Gunze Co., Ltd.) were used as scaffold materials, and each cut to obtain a test piece having a size of 10 mm × 10 mm × 3 mm. Then, the respective scaffold materials were combined with fat precursor cells derived from man and gelatin particles soaked with a basic fibroblast growth factor, in order to regenerate the fat under the back skin of a mouse.

First of all, the fat precursor cells derived from man were obtained by isolation from fat tissue sampled during excising a breast cancer from a patient obtaining informed consent in Kyoto University in the following way. Specifically, the aforementioned fat tissue was washed with PBS (phosphoric acid buffer solution) and treated with collagenase (produced by Nitta Gelatin) at 37 °C for 20 minutes. Thereafter, a culture medium (Medium 199) containing a 10 weight % fetal bovina serum was added thereto, and the resultant mixture was separated with a centrifugal separator (for 5 minutes per 200 g) to remove the resultant supernatant, and the same procedure of adding the culture medium containing the fetal bovina serum and separating with the centrifugal separator was further repeated twice. Subsequently, a culture medium as obtained by adding a 0.1 µg/ml basic fibroblast growth factor was added thereto, and the resultant mixture was cultured in a cell-culture flask (75 cm², CORNING43072, 1 × 10³ cells/cm²) in the presence of 5 % CO₂ at 37 °C for 7 days, thus obtaining the fat precursor cells derived from man.

On the one hand, the gelatin particles soaked with the basic fibroblast growth factor were obtained by adding 1 µg (50 µl) of the basic fibroblast growth factor to 2 mg of gelatin particles which were produced by subjecting a gelatin solution in the form of an emulsion to glutaraldehyde-crosslinking, and thereafter leaving the resultant mixture at room temperature for 1 hour to bind the gelatin particles with the basic fibroblast growth factor.

Next, the fat precursor cells derived from man with a cell density of 1 × 10⁵ cells/culture medium (50 µl) were mixed with the above gelatin particles soaked with the basic fibroblast growth factor in order to obtain a suspension. Then, the above suspension was added to a scaffold material, and the resultant mixture was incubated for 3 hours.

The respective scaffold materials (the material of Example 6 and the simple material consisting of collagen) were combined with the fat precursor cells derived from man and the gelatin particles soaked with the basic fibroblast growth factor, as obtained in this way, and the resultant combined materials were used as samples. Nude mice (BALB/c, 6w, ♀) were separately cut at a back portion partially. Therefrom, both of these samples were each buried under the back skin of a mouse, and thereafter the portion was sutured. Then, they were sacrificially killed after 42 days, and thereafter the back portion was cut and opened to observe the sample.

A view obtained by digital camera photography of the sample including the simple material consisting of collagen and a view obtained by digital camera photography of the sample including the material of Example 6 were shown in Figs. 17A and 17B, respectively. From the result of Fig. 17, as to both samples, it could be confirmed that blood vessels intruded into the samples and fatty tissue was formed.

In addition, when the residual volume of both samples was measured, the residual volume of the sample including the material of Example 6 was 36.9 mm³, but that of the sample including the simple material consisting of collagen was 26.8 mm³. These results are graphed in Fig. 18. From this result, it was apparent that the residual volume of the sample including the material of Example 6 is significantly larger than that of the sample including the simple material consisting of collagen. This shows that: the low mechanical strength of the collagen sponge is enhanced by comprising the poly(glycolic acid) fiber, and the excellent mechanical properties can be given.

From the above result of the fat regeneration test under the back skin of a mouse, it was apparent that: the poly(glycolic acid) fiber improved the material strength without damaging the cell affinity of the collagen, and the residual fiber did not hinder the regeneration of fat tissue because its amount as used was small. As a result, it showed that the material operated as a scaffold for regeneration of tissue having much larger volume and significantly increased the volume of the regenerated fat tissue. Incidentally, it is also known that the biodegradation absorbability of the collagen itself is identical whether the poly(glycolic acid) fiber exists or not.

### INDUSTRIAL APPLICATION

The present invention can provide a hybrid material for regeneration of living body tissue, which is excellent in affinity with cells, and which can retain sufficient mechanical strength for a term needed to regenerate the tissue, and which simultaneously has moderate biodegradation absorbability which enables degradation and disappearance rapidly after the regeneration of the tissue.

## Claims

1. A hybrid material for regeneration of living body tissue comprising (A) a sponge including a crosslinked naturally-occurring polymer and (B) biodegradation-absorbable synthetic polymer fibers, said hybrid material being **characterized in**
**that** said biodegradation-absorbable synthetic polymer fibers exist in the sponge,
**that** said biodegradation-absorbable synthetic polymer fibers are coated with a biodegradation-absorbable synthetic polymer having a melting point lower than the melting point of the biodegradation-absorbable synthetic polymer fibers for flocculating at an intersecting point, and
**that** the weight ratio of biodegradation-absorbable synthetic polymer/naturally-occurring polymer is in the range of 0.2 to 10.0.

2. A hybrid material for regeneration of living body tissue according to claim 1, wherein the biodegradation-absorbable synthetic polymer fiber is selected from the group consisting of poly(glycolic acid), poly(lactic acid), poly-ε-caprolactone, polydioxanone, poly(β-malic acid), poly(ortho esters), polydiaminophosphazene, and copolymers thereof.

## Patentansprüche

1. Hybridmaterial zur Regeneration von lebendem Körpergewebe, das (A) einen Schwamm, der ein vernetztes, natürlich auftretendes Polymer einschließt, und (B) Bioabbau-absorbierbare synthetische Polymerfasern umfasst, wobei das Hybridmaterial **dadurch gekennzeichnet ist,**
**dass** die Bioabbau-absorbierbaren synthetischen Polymerfasern in dem Schwamm vorhanden sind,
**dass** die Bioabbau-absorbierbaren synthetischen Polymerfasern mit einem Bioabbau-absorbierbaren synthetischen Polymer beschichtet sind, das einen Schmelzpunkt aufweist, der niedriger als der Schmelzpunkt der Bioabbau-absorbierbaren synthetischen Polymerfasern, zur Flockung an einem Überschneidungspunkt, ist und
**dass** das Gewichtsverhältnis von Bioabbau-absorbierbarem synthetischen Polymer/natürlich auftretendem Polymer im Bereich von 0,2 bis 10,0 liegt.

2. Hybridmaterial zur Regeneration von lebendem Körpergewebe gemäß Anspruch 1, wobei die Bioabbau-absorbierbare synthetische Polymerfaser ausgewählt ist aus der Gruppe bestehend aus Polyglycolsäure, Polymilchsäure, Poly-ε-caprolacton, Polydioxanon, Poly-β-äpfelsäure, Polyorthoester, Polydiaminophosphazen und deren Copolymeren.

## Revendications

1. Matériau hybride pour la régénération d'un tissu corporel vivant comprenant (A) une éponge comprenant un polymère survenant à l'état naturel réticulé et (B) des fibres de polymère synthétique absorbable par biodégradation, ledit matériau hybride étant **caractérisé en ce que**
lesdites fibres de polymère synthétique absorbable par biodégradation existent dans l'éponge,
lesdites fibres de polymère synthétique absorbable par biodégradation sont revêtues d'un polymère synthétique absorbable par biodégradation ayant un point de fusion inférieur au point de fusion des fibres de polymère synthétique absorbable par biodégradation pour permettre une floculation à un point d'intersection, et
le rapport en poids du polymère synthétique absorbable par biodégradation/polymère survenant à l'état naturel est compris dans la plage de 0,2 à 10,0.

2. Matériau hybride pour la régénération d'un tissu corporel vivant selon la revendication 1, dans lequel la fibre de polymère synthétique absorbable par biodégradation est sélectionnée dans le groupe consistant en l'acide polyglycolique, l'acide polylactique, la poly-ε-caprolactone, le polydioxanone, le poly(acide β-malique), des polyorthoesters, le polydiaminophosphazène, et des copolymères de ceux-ci.
